# EUROPÄISCHE PATENTANMELDUNG

(11) **EP 4 389 974 A2**
(43) Veröffentlichungstag der Anmeldung: **26.06.2024**
(21) Anmeldenummer: 23186290.5
(22) Anmeldetag: 19.07.2023
(51) Int. Cl.: D21J 3/10, D21J 7/00

(54) **VORRICHTUNG UND VERFAHREN ZUR GLEICHZEITIGEN HERSTELLUNG VON MEHREREN FASERN UMFASSENDEN BEHÄLTERN**

(30) Priorität: 20.12.2022 DE 102022134094
(71) Anmelder: KRONES Aktiengesellschaft, 93073 Neutraubling (DE)
(72) Erfinder: WINZINGER, Frank, Neutraubling (DE); ALBRECHT, Thomas, Neutraubling (DE); PETER, Daniel, Neutraubling (DE); MEYER, Andreas, Neutraubling (DE); HANDSCHUH, Eduard, Neutraubling (DE); HIPPEL, Andreas, Neutraubling (DE)
(74) Vertreter: Grünecker Patent- und Rechtsanwälte PartG mbB

(57) **Zusammenfassung**

Die Erfindung betrifft eine Vorrichtung (1, 41) zur gleichzeitigen Herstellung von mehreren Fasern umfassenden Behältern unter der Verwendung von faserhaltiger Pulpe. Weiter betrifft die Erfindung ein Verfahren zur gleichzeitigen Herstellung von mehreren Fasern umfassenden Behältern unter der Verwendung von faserhaltiger Pulpe mittels der Vorrichtung (1, 41).

## Beschreibung

Die Erfindung betrifft eine Vorrichtung und ein Verfahren zur gleichzeitigen Herstellung von mehreren Fasern umfassenden Behältern gemäß den unabhängigen Ansprüchen.

### Stand der Technik

Es ist üblich, Fasern umfassende Behälter, beispielsweise unter der Verwendung von Pulpe, die die Fasern umfasst, in porösen Formen herzustellen. Die Pulpe wird in die Form eingebracht und durch Aufblasen eines in die Form eingebrachten elastischen Ballons, der die Pulpe gegen die Form drückt, kann ein Fasern umfassender Behälter ausgeformt werden. Bei dieser Herstellungsweise können sich lange Zykluszeiten ergeben.

### Aufgabe

Die Aufgabe der Erfindung ist es, eine Vorrichtung und ein Verfahren zur gleichzeitigen Herstellung von mehreren Fasern umfassenden Behältern zur Verfügung zu stellen, die ein kontinuierliches, gleichzeitiges Fertigformen mehrere Fasern umfassender Behälter ermöglichen.

### Lösung

Die Aufgabe wird gelöst durch die Vorrichtung und das Verfahren zur gleichzeitigen Herstellung von mehreren Fasern umfassenden Behältern gemäß den unabhängigen Ansprüchen. Weitere Ausführungsformen sind in den Unteransprüchen offenbart.

Die erfindungsgemäße Vorrichtung zur gleichzeitigen Herstellung von mehreren Fasern umfassenden Behältern unter der Verwendung von faserhaltiger Pulpe.

Die gleichzeitige Herstellung kann eine zeitlich gleichzeitige Herstellung mittels der Vorrichtung bedeuten.

Die Fasern können Lignin, Bananenblätter und/oder Chinin umfassen. Die Fasern können beispielsweise Zellulosefasern, Fasern von Nadelhölzern, Blattgehölzen und/oder Platanen und/oder von Gräsern, Schilf und/oder Bambus oder dergleichen umfassen. Die Fasern können Seidenfäden, Spinnenfäden, Algen, natürliche Fasern (wie Donau-Silphie-Fasern, Hanf, Mais, Baumwolle), Bananenschalen, Orangenschalen, Gras, Stroh, Kartoffelstärke oder aufbereiteten Kuhdung umfassen. Auch können Zellstofffasern vorgesehen sein, die einem Prozess entstammen, durch den sie künstlich gezüchtet wurden. Diese alternativen Materialien können bei Materialengpässen von Holz als Grundstoff für eine fluide Masse mit Fasern, den Grundstoff vollständig oder teilweise ersetzen.

Die Fasern können Fasermischungen aus holzfremden Material, beispielsweise Baumwolle, Hanf, und/oder Textilfasern umfassen.

Beispielsweise können die Fasern Viskosefasern umfassen. Eine Viskosefaser ist eine künstliche Faser aus regenerierter Zellulose, wobei die Viskosefasern als Ausgangsbasis 100% Zellulose haben, die in einem mehrstufigen Verfahren behandelt wird. Die Viskosefasern können eine flache oder kabelförmige Struktur, eine trilobale Form oder eine doppelt trilobale Struktur umfassen. Die Fasern können eine flächige oder hohle Struktur oder eine geriffelte oder raue äußere Oberfläche umfassen.

Es können auch verschiedenartige Fasern in Kombination verwendet werden. Beispielsweise können Fasern mit unterschiedlichen äußeren Oberflächen und/oder Formen und/oder hohler Struktur und/oder flächiger Struktur und/oder aus verschiedenen Materialien kombiniert werden.

Durch den natürlichen Ursprung der Fasern können diese biologisch abbaubar sein. Zudem sind sie nachhaltig und nachwachsend.

Die Fasern können in einer Pulpe, die beispielsweise zum Herstellen des Fasern umfassenden Behälters verwendet werden kann, verwendet werden. Die Pulpe kann eine Mischung aus Wasser, beispielsweise mit Additiven, und Fasern sein oder umfassen. Die Pulpe kann eine Mischung aus Fasern und flüssigen Bestandteilen umfassen.

Die Behälter können Flaschen, Dosen oder Becher sein oder umfassen.

Die Vorrichtung kann mehrere Stationen zum jeweiligen Ausformen eines Fasern umfassenden Behälters umfassen. Die mehreren Stationen können an einer Rundläufermaschine angeordnet sein.

Jede Station kann eine Vorform und eine Pressform umfassen, wobei beispielsweise die Vorform eine Gitterstruktur umfassen kann. Die Vorform und/oder die Pressform können jeweils eine Negativform einer Außenkontur des herzustellenden Behälters umfassen. Die Vorform kann für ein Vorformen des herzustellenden Behälters verwendet werden. In der Pressform kann ein in der Vorform vorgeformter Behälter gepresst werden, beispielsweise um zumindest einen Teil des Wassers der Pulpe herauszupressen und/oder um die Fasern der Pulpe ineinander zu pressen.

Die Vorrichtung kann zwei Untervorrichtungen umfassen, wobei die erste Untervorrichtung Vorformen umfassen kann und die zweite Untervorrichtung Pressformen umfassen kann. Beide Untervorrichtungen können jeweils als Rundläufer mit mehreren Stationen ausgebildet sein. Beispielsweise kann die Vorrichtung und/oder eine oder beide Untervorrichtungen eine Vielzahl von Stationen mit Formen umfassen, wobei beispielsweise die Stationen in äquidistanten Abständen entlang des Umfangs eines sich taktweise oder kontinuierlich drehenden Rades (Rundläufers) angeordnet sein können.

Die Vorrichtung kann weiter eine Vorrichtung zum Zuführen von faserhaltiger Pulpe umfassen. Die Vorrichtung kann beispielsweise einen Drehverteiler umfassen. Beispielsweise kann der Drehverteiler Pulpe und/oder Fasern von einem stationären Teil der Vorrichtung zu den Stationen im drehenden Teil führen. Im Fall von zwei Untervorrichtungen, kann ein Drehverteiler für Pulpe und/oder Fasern nur bei der ersten Untervorrichtung vorhanden sein.

Es können auch mehrere Drehverteiler oder Drehverteilerspuren (z.B. konzentrisch im selben Drehverteiler) vorgesehen sein, wobei beispielsweise zusätzlich zu der Pulpe und/oder den Fasern in einer weiteren Spur oder einem weiteren Drehverteiler Druckluft zu den Stationen transportiert werden kann. Im Fall von zwei Untervorrichtungen kann die erste und/oder die zweite Untervorrichtung einen Druckluftdrehverteiler umfassen.

Ein Druckluftdrehverteiler kann auf seiner stationären Seite mit einem Kompressor verbunden sein. Der Kompressor kann von der Vorrichtung umfasst sein oder unabhängig von der Vorrichtung zur Verfügung gestellt werden.

Eine Verrohrung des Drehverteilers kann derart ausgebildet sein, dass die faserhaltige Pulpe durch die Rohrleitungen passieren kann, beispielsweise ohne diese zu verstopfen.

Die Vorrichtung kann weiter eine Vorrichtung zum Sammeln und Rückführen zumindest eines Teils von Wasser, das in der faserhaltigen Pulpe umfasst ist, umfassen. Der Teil des Wassers kann in der Vorform und/oder in der Pressform aus der Pulpe entfernt werden, beispielsweise durch Pressen und/oder durch auf den herzustellenden Behälter einwirkende Fliehkräfte.

Die Vorrichtung kann weiter mindestens eine Übertragungsvorrichtung von Presskraft auf mindestens zwei der Pressformen umfassen. So kann nur eine Übertragungsvorrichtung für mehrere Pressformen erforderlich sein und nicht beispielsweise für jede Pressform jeweils eine zugeordnete Übertragungsvorrichtung.

Die mindestens eine Übertragungsvorrichtung kann einen Motor und mindestens einen Hebel umfassen, beispielsweise mindestens einen Kniehebel, der durch den Motor antreibbar sein kann, wodurch auf die mindestens zwei der Pressformen ein Pressdruck ausübbar sein kann. Mittels des Hebels kann von außen eine Presskraft auf die Pressform ausgeübt werden.

Die mindestens eine Übertragungsvorrichtung kann eine Bereitstellungsvorrichtung für Druckluft umfassen, wobei die Bereitstellungsvorrichtung mit mindestens zwei elastischen Ballonen (manchmal auch Blasen genannt) mittels eines Rohrleitungssystems zum Verteilen der Druckluft verbunden sein kann, wobei jeder der elastischen Ballone vorgesehen sein kann zum Einbringen in eine der Pressformen und zum Aufblasen mittels der Druckluft.

Die mindestens zwei elastischen Ballone können von der Übertragungsvorrichtung umfasst sein. Mittels der Ballone kann von innen eine Presskraft auf die Pressform ausgeübt werden.

Die Druckluft eines Ballons einer Station kann nach dem Pressen zumindest teilweise zu einem Aufbau von Druck im Ballon einer anderen Station wiederverwendet werden. Hierfür können die Stationen untereinander mit Leitungen verbunden sein.

Die Vorrichtung kann ausgebildet sein, bei der gleichzeitigen Herstellung der mehreren Fasern umfassenden Behälter eine trennbare Verbindung der mehreren Behälter unter Verwendung der faserhaltigen Pulpe vorzusehen. Beispielsweise kann die trennbare Verbindung eine oder mehrere Perforationen umfassen. Beispielsweise kann die trennbare Verbindung Stege und/oder eine durchgehende flächige Verbindung zwischen den mehreren Behältern umfassen.

Beispielsweise kann ein Sechser-Pack von Behältern vorgesehen sein, die beispielsweise in einer Matrix von 2 × 6 oder 2 x 3 angeordnet sein können. Mittels der trennbaren Verbindung kann einer der Behälter von den restlichen Behältern getrennt werden, wobei die Verbindung zwischen den restlichen Behältern erhalten bleiben kann.

Die Vorrichtung kann weiter eine Entnahmevorrichtung zum Entnehmen der Fasern umfassenden Behälter aus den Vor- bzw. Pressformen umfassen. Beispielsweise kann die Vorrichtung weiter eine Füllvorrichtung zum gleichzeitigen Befüllen der mehreren Behälter mit Produkt (z.B. ein fluides Produkt, wie ein Getränk oder dergleichen) umfassen. Beispielsweise kann die Vorrichtung weiter eine Verschließvorrichtung zum gleichzeitigen Verschließen der mehreren Behälter umfassen. Beispielsweise kann die Vorrichtung weiter eine Etikettiervorrichtung zum gleichzeitigen oder aufeinanderfolgenden Etikettieren der Behälter umfassen.

Die Vorrichtung kann zum Zuführen von faserhaltiger Pulpe einen Drehverteiler umfassen. Eine Verrohrung des Drehverteilers kann derart ausgebildet sein, dass die faserhaltige Pulpe durch die Rohrleitungen passieren kann, beispielsweise ohne diese zu verstopfen.

Die Vorrichtung zum Sammeln und Rückführen zumindest eines Teils von Wasser, das in der faserhaltigen Pulpe umfasst sein kann, kann in Wirkrichtung der Schwerkraft unterhalb der mehreren Stationen angeordnet sein. Der zumindest eine Teil des Wassers kann dann unter Einwirkung der Schwerkraft in die Vorrichtung zum Sammeln und Rückführen tropfen und/oder fließen. Beispielsweise kann die Vorrichtung zum Sammeln und Rückführen eine Wanne, beispielsweise eine ringförmige Wanne, beispielsweise als Wasserschloss ausgebildet, umfassen. Beispielsweise kann zudem in Wirkrichtung der Schwerkraft unterhalb der mehreren Stationen eine zur Wanne geneigte Mantelfläche eines Kreiskegels vorgesehen sein. Der zumindest eine Teil des Wassers kann dann unter Einwirkung der Schwerkraft entlang der Mantelfläche fließen und in die Wanne gelangen.

Die Vorrichtung kann eine separate Drehverteilerspur oder einen separaten Drehverteiler für das Rückführen von Auspressmedium umfassen. Das Auspressmedium kann Wasser und/oder Anteile von Fasern und/oder sonstige Bestandteile der Pulpe umfassen, die beispielsweise beim Pressen der Pulpe bzw. beim Ausformen des Fasern umfassenden Behälters ausgepresst werden und/oder sich dabei lösen können.

Alternativ kann eine Abtropfrinne unterhalb der Stationen vorgesehen sein, die als "Drehverteiler" dienen kann und in der Flüssigkeit und/oder das Auspressmedium gesammelt und rückgeführt werden kann.

Ein Drehverteiler für das Rückführen von Auspressmedium, beispielsweise eine separate Drehverteilerspur oder ein separater Drehverteiler, kann auf seiner stationären Seite mit einem Ausmischbehälter verbunden sein.

Die Vorrichtung zum Sammeln und Rückführen zumindest eines Teils von Wasser, das in der faserhaltigen Pulpe umfasst ist, kann den Drehverteiler umfassen.

Die Vorrichtung kann weiter eine Vorrichtung zum Herstellen der faserhaltigen Pulpe umfassen, wobei die Vorrichtung zum Herstellen der faserhaltigen Pulpe mit der Vorrichtung zum Sammeln und Rückführen zumindest eines Teils von Wasser, das in der faserhaltigen Pulpe umfasst ist, durch eine Zuführleitung leitungsverbunden sein kann zum Zuführen des zumindest einen Teils von Wasser, das in der faserhaltigen Pulpe umfasst ist, und zum Verwenden des zumindest einen Teils von Wasser, das in der faserhaltigen Pulpe umfasst ist, für die Herstellung weiterer faserhaltiger Pulpe. Das ausgepresste Wasser, das dem zumindest eines Teils von Wasser, das in der faserhaltigen Pulpe umfasst ist, entsprechen kann, kann so in einem Kreislauf gefahren werden. Der Wasserverbrauch kann verringert werden. Durch das Zuführen des zumindest einen Teils von Wasser, das in der faserhaltigen Pulpe umfasst ist, zu der Vorrichtung zum Herstellen der faserhaltigen Pulpe kann ein Führen dieses Wassers entlang einer Wasserkaskade der Vorrichtung zur gleichzeitigen Herstellung von mehreren Fasern umfassenden Behältern unter der Verwendung von faserhaltiger Pulpe vermieden werden. Dieses Wasser kann als ein mehr oder weniger definiertes Volumen direkt bei der Vorrichtung verbleiben. Die Vorrichtung zum Herstellen der faserhaltigen Pulpe kann eine Heizvorrichtung für die faserhaltige Pulpe umfassen.

Die Zuführleitung kann eine Desinfektionsvorrichtung umfasst zum Desinfizieren des zumindest einen Teils von Wasser, das in der faserhaltigen Pulpe umfasst ist, wobei beispielsweise die Desinfektionsvorrichtung ausgebildet ist zum Ausbringen eines Desinfektionsmittels in den zumindest einen Teils von Wasser, das in der faserhaltigen Pulpe umfasst ist, und/oder zum Ausbringen von UV-A-, UV-B- und/oder UV-C-Strahlung zum Bestrahlen des zumindest einen Teils von Wasser, das in der faserhaltigen Pulpe umfasst ist.

Die Vorrichtung kann weiter eine Regelungsvorrichtung zum Messen einer Qualität des zumindest einen Teils von Wasser, das in der faserhaltigen Pulpe umfasst ist, und zum Regeln der Desinfektionsvorrichtung entsprechend eines Ergebnisses der Messung der Qualität umfassen.

Die Vorrichtung kann ein rundlaufendes Karussell umfassen und die Stationen können an einem Umfang des rundlaufenden Karussells vorgesehen sein. Das rundlaufende Karussell kann für ein Anströmen der faserhaltigen Pulpe in der Vorform und Pressform ausgebildet sein. Beispielsweise kann jeder der Vorformen und Pressformen ein Einlassventil zugeordnet sein. Beispielsweise kann eine Heizvorrichtung für die Vorformen und Pressformen vorgesehen sein. Dieses rundlaufende Karussell kann als ein erstes Karussell angesehen werden, wenn wie weiter unten beschrieben ein bzw. zwei weitere umlaufende Karusselle vorgesehen werden können. Beispielsweise kann die faserhaltige Pulpe, die zum Anströmen verwendet werden kann, erhitzt sein. Das hier erwähnte rundlaufende Karussell kann auch als erstes rundlaufendes Karussell bezeichnet werden für den Fall, dass mehr als nur ein rundlaufendes Karussell vorgesehen ist. Durch die Einlassventile kann die Anströmung während der Rotation gesteuert werden.

Das Anströmen der faserhaltigen Pulpe in der Vorform und Pressform kann von unten nach oben erfolgen, also beispielsweise entgegen der Wirkrichtung der Schwerkraft. Beim Anströmen von unten nach oben kann vorgesehen sein, dass die Vorform und Pressform derart angeordnet sein kann, dass der hergestellte Behälter dann kopfüber in der Vorform und Pressform angeordnet ist.

Ein rundlaufendes Karussell, wie auch die im Weiteren genannten rundlaufenden Karusselle (zur Unterscheidung beispielsweise mit "zweites", "drittes" bezeichnet), können einen geschlossenen Transportpfad umfassen. Der Transportpfad kann neben nichtlinearen Abschnitten einen oder mehrere lineare Abschnitte umfassen. Der Transportpfad kann bei einer Draufsicht oval oder kreisförmig oder nierenförmig verlaufen.

Die Vorrichtung kann ein zweites rundlaufendes Karussell umfassen, das stromab des rundlaufenden Karussells angeordnet sein kann, wobei das zweite rundlaufende Karussell für eine Innentrocknung von in der Vorform und Pressform angeströmten faserhaltigen Pulpe ausgebildet sein kann. Die Innentrocknung kann mittels warmer Luft erfolgen.

Die Vorrichtung kann ein drittes rundlaufendes Karussell umfassen, das stromab des rundlaufenden Karussells angeordnet sein kann, wobei das dritte rundlaufende Karussell für ein Pressen von in der Vorform und Pressform angeströmten faserhaltigen Pulpe und für ein Aufbringen einer Innenbeschichtung ausgebildet sein kann. Beispielsweise kann das dritte rundlaufende Karussell in einem aseptischen Raum angeordnet sein. Nach dem Aufbringen der Innenbeschichtung kann eine weitere Trocknung von außen erfolgen.

Es kann vorgesehen sein, dass in einer Transportrichtung der Vorformen und Pressformen dem umlaufenden Karussell das zweite umlaufende Karussell und dem zweiten umlaufenden Karussell das dritte umlaufende Karussell nachfolgt. Alternativ kann vorgesehen sein, dass in einer Transportrichtung der Vorformen und Pressformen dem umlaufenden Karussell das dritte umlaufende Karussell und dem dritten umlaufenden Karussell das zweite umlaufende Karussell nachfolgt. Es kann alternativ auch vorgesehen sein, dass nur das umlaufende Karussell und das dritte Karussell vorgesehen sind und das zweite Karussell in dieser Ausführungsform nicht vorgesehen ist. Die Bezeichnung "drittes" rundlaufendes Karussell wird hier beibehalten (auch wenn es insgesamt nur zwei umlaufende Karusselle sind), um die Merkmale, die diesem umlaufenden Karussell zugeordnet worden sind, weiterhin eindeutig zuordnen zu können.

Zwischen den Karussellen können jeweils ein oder mehrere Übergabesterne vorgesehen sein. Ein Übergabestern kann als Teilungsverzugsstern ausgebildet sein.

Das rundlaufende Karussell kann weiter für ein Pressen von in der Vorform und Pressform angeströmten faserhaltigen Pulpe ausgebildet ist. Das Pressen kann mittels gasförmigen Medien und einem Überdruck erfolgen.

Die Vorrichtung kann weiter eine Rückführvorrichtung zum Rückführen der Vorformen und Pressformen nach Entnahme von Fasern umfassenden Behälter zu dem rundlaufenden Karussell umfassen. Die Fasern umfassenden Behälter können in der einen Ausführungsform an einer Entnahmeposition des dritten umlaufenden Karussells aus den Vorformen und Pressformen entnommen werden und können in der anderen Ausführungsform an einer Entnahmeposition des zweiten umlaufenden Karussells aus den Vorformen und Pressformen entnommen werden. Dazu können die Vorformen und Pressformen geöffnet werden. Die Fasern umfassenden Behälter können nach dem Entnehmen weiteren Elementen der Vorrichtung zugeführt werden, um dort weiteren Behandlungsschritten unterzogen werden zu können.

Die Vorrichtung kann weiter eine Reinigungsvorrichtung und eine Inspektionsvorrichtung für mittels der Rückführvorrichtung rückgeführten Vorformen und Pressformen umfassen. Bei einer Rückführung können sich die Vorformen und Pressformen in einem geöffneten Zustand befinden, den sie beispielsweise nach einem Öffnen an der Entnahmeposition des dritten umlaufenden Karussells bzw. des zweiten umlaufenden Karussells haben. Beispielsweise kann die Inspektionsvorrichtung eine Ausschleusvorrichtung für defekte Vorformen und Pressformen umfassen. Die Vorrichtung kann eine Trocknungsvorrichtung für ein Trocknen von außen der aus den Vorformen und Pressformen entnommenen Fasern umfassenden Behälter, eine Füll- und Verschließvorrichtung und eine Etikettiervorrichtung umfassen. Beispielsweise können die Trocknungsvorrichtung und die Füll- und Verschließvorrichtung in einem aseptischen Raum angeordnet sein. Dieser aseptische Raum kann auch das dritte rundlaufende Karussell umfassen. Für das Trocknen von außen kann der Fasern umfassende Behälter von innen vorgespannt sein.

Die Vorrichtung kann derart ausgebildet sein, dass die Fasern umfassenden Behälter derart anordenbar sein können, dass eine Längsachse der Behälter zu einer Wirkrichtung der Schwerkraft einen Winkel zwischen 70° bis 110° einschließen kann. Die Behälter können als liegend angenommen werden.

Die Vorrichtung kann modular aufgebaut sein.

Die Vorrichtung kann eine Beschichtungsvorrichtung umfassen, die ausgebildet sein kann, die Behälter nach dem Ausformen zu beschichten, beispielsweise mit einem Wachs. Die Behälter können durch die Beschichtung undurchlässig für im Behälter befindliches Produkt sein. Die Beschichtungsvorrichtung kann einen separaten Rundläufer mit einer Vielzahl an Beschichtungsstationen umfassen.

Für den Transport von Pulpe können Pumpen von der Vorrichtung umfasst sein.

Für den Fall, dass die Behälter aus trockenen Fasern gepresst bzw. ausgeformt werden, können zum Transport der Fasern Gebläse, Schnecken, Schieber oder dgl. von der Vorrichtung umfasst sein.

Die Vorrichtung kann ein Langstator-Linearmotor-System umfassen, das eine Vielzahl von Movern umfassen kann, die jeweils mindestens einen Behälter von der Pressstation zu einer stromabwärts angeordneten Station (z.B. Beschichtungsstation oder Füllstation) transportieren können.

Weiter ist ein Verfahren zur gleichzeitigen Herstellung von mehreren Fasern umfassenden Behältern unter der Verwendung von faserhaltiger Pulpe mittels der Vorrichtung, wie oben oder weiter unten beschrieben, vorgesehen.

Mittels der Vorrichtung zum Zuführen von faserhaltiger Pulpe, beispielsweise einem Drehverteiler, kann die Pulpe den mehreren Stationen zum jeweiligen Ausformen eines Fasern umfassenden Behälters zugeführt werden. Jede der Stationen kann eine Vorform und eine Pressform umfassen. Mittels der mindestens einen Übertragungsvorrichtung von Presskraft auf mindestens zwei der Pressformen kann eine Presskraft auf die in den Stationen vorhandene Pulpe ausgeübt werden.

Ein dabei ausgepresster Teil von Wasser, das in der Pulpe umfasst sein kann, kann mittels der Vorrichtung zum Sammeln und Rückführen, die in Wirkrichtung der Schwerkraft unterhalb der mehreren Stationen angeordnet sein kann, gesammelt und rückgeführt werden.

Der ausgepresste Teil von Wasser (auch bezeichnet als der zumindest eine Teil von Wasser, das in der faserhaltigen Pulpe umfasst ist) kann durch eine Zuführleitung, die die Vorrichtung mit der Vorrichtung zum Herstellen der faserhaltigen Pulpe leitungsverbindet, rückgeführt und für die Herstellung weiterer faserhaltiger Pulpe verwendet werden. Die faserhaltige Pulpe (auch die weitere faserhaltige Pulpe) kann mittels der Heizvorrichtung, die von der Vorrichtung zum Herstellen der faserhaltigen Pulpe umfasst sein kann, erhitzt werden.

Die Zuführleitung kann eine Desinfektionsvorrichtung umfassen zum Desinfizieren. Der zumindest eine Teil von Wasser, das in der faserhaltigen Pulpe umfasst ist, kann mittels der Desinfektionsvorrichtung desinfiziert werden.

Mittels der Regelungsvorrichtung kann eine Qualität des zumindest einen Teils von Wasser, das in der faserhaltigen Pulpe umfasst ist, gemessen werden und die Desinfektionsvorrichtung kann entsprechend eines Ergebnisses der Messung der Qualität geregelt werden.

Mittels des rundlaufenden Karussells kann faserhaltige Pulpe in den Vorformen und Pressformen angeströmt werden.

Mittels des zweiten rundlaufenden Karussells kann eine Innentrocknung von in der Vorform und Pressform angeströmten faserhaltigen Pulpe erfolgen.

Mittels des dritten rundlaufenden Karussells können ein Pressen von in der Vorform und Pressform angeströmten faserhaltigen Pulpe und ein Aufbringen einer Innenbeschichtung erfolgen.

Ein Rückführen der Vorformen und Pressformen nach Entnahme von Fasern umfassenden Behälter zu dem rundlaufenden Karussell kann mittels der Rückführvorrichtung erfolgen.

Die Fasern umfassenden Behälter können in der einen Ausführungsform an einer Entnahmeposition des dritten umlaufenden Karussells aus den Vorformen und Pressformen entnommen werden und können in der anderen Ausführungsform an einer Entnahmeposition des zweiten umlaufenden Karussells aus den Vorformen und Pressformen entnommen werden. Dazu können die Vorformen und Pressformen geöffnet werden.

Mittels der Reinigungsvorrichtung kann eine Reinigung und mittels der Inspektionsvorrichtung kann eine Inspektion für mittels der Rückführvorrichtung rückgeführten Vorformen und Pressformen erfolgen. Mittels der Ausschleusvorrichtung der Inspektionsvorrichtung können defekte Vorformen und Pressformen ausgeschleust werden.

Mittels der Trocknungsvorrichtung kann ein Trocknen von außen der aus den Vorformen und Pressformen entnommenen Fasern umfassenden Behälter erfolgen. Mittels der Füll- und Verschließvorrichtung kann ein Füllen der Fasern umfassenden Behälter mit Produkt und ein Verschließen der befüllten Behälter erfolgen. Mittels der Etikettiervorrichtung können die befüllten und verschlossenen Fasern umfassenden Behälter etikettiert werden.

Beispielsweise kann bei der gleichzeitigen Herstellung der mehreren Fasern umfassenden Behälter eine trennbare Verbindung der mehreren Behälter unter Verwendung der faserhaltigen Pulpe vorgesehen werden.

Die mehreren Behälter können mittels einer Füllvorrichtung zum gleichzeitigen Befüllen der mehreren Behälter gleichzeitig mit Produkt befüllt werden. Die mehreren mit Produkt befüllten Behälter können mit der Verschließvorrichtung gleichzeitig verschlossen werden. Die mehreren verschlossenen Behälter können mit der Etikettiervorrichtung gleichzeitig oder nacheinander etikettiert werden.

Die Vorrichtung kann weiter einen Zentralkessel oder eine Ringleitung umfassen, die derart ausgebildet sein können, dass beim Anströmen faserhaltige Pulpe aus dem Kessel oder aus der Ringleitung zu den einzelnen Vorformen und Pressformen leitbar ist.

Weiter kann die Vorrichtung einen Füllkessel, beispielsweise zum Bereitstellen von faserhaltiger Pulpe umfassen, der derart ausgebildet sein kann, dass ein Füllstand im Füllkessel auf einem gegebenen Wert haltbar und ein Prozessdruck regelbar ist.

Die Vorformen und Pressformen können beheizbar ausgebildet sein. Beispielsweise kann eine elektrische Heizung vorgesehen sein oder es kann ein erhitztes Medium, wie beispielsweise Öl verwendet werden, das beispielsweise durch Medienkanäle der Vorformen und Pressformen leitbar sein kann. Durch die erhitzten Vorformen und Pressformen kann ein Trocknungsprozess von angeströmter und/oder ausgepresster faserhaltiger Pulpe in den Vorformen und Pressformen eingeleitet werden, beispielsweise vor einem nachfolgenden, separaten Trocknungsvorgang.

Auch durch die Verwendung von erhitzter, faserhaltiger Pulpe in erhitzten Vorformen und Pressformen oder in nicht-erhitzten Vorformen und Pressformen kann ein Trocknungsprozess von angeströmter und/oder ausgepresster faserhaltiger Pulpe in den Vorformen und Pressformen eingeleitet werden, beispielsweise vor einem/dem nachfolgenden, separaten Trocknungsvorgang.

Der nachfolgenden, separaten Trocknungsvorgang kann mittels einer nachfolgenden Trocknungsvorrichtung erfolgen, die von der Vorrichtung umfasst sein kann.

### Kurze Figurenbeschreibung

Die beigefügten Figuren stellen beispielhaft zum besseren Verständnis und zur Veranschaulichung Aspekte und/oder Ausführungsbeispiele der Erfindung dar. Es zeigt:
Figur 1 eine Schnittdarstellung der Vorrichtung zur gleichzeitigen Herstellung von mehreren Fasern umfassenden Behältern unter der Verwendung von faserhaltiger Pulpe,
Figur 2 eine Schnittdarstellung einer ersten Ausführungsform einer Übertragungsvorrichtung von Presskraft auf zwei Pressformen,
Figur 3 eine Schnittdarstellung einer zweiten Ausführungsform einer Übertragungsvorrichtung von Presskraft auf vier Pressformen und
Figur 4 eine Draufsicht auf eine Vorrichtung zur gleichzeitigen Herstellung von mehreren Fasern umfassenden Behältern unter der Verwendung von faserhaltiger Pulpe.

### Ausführliche Figurenbeschreibung

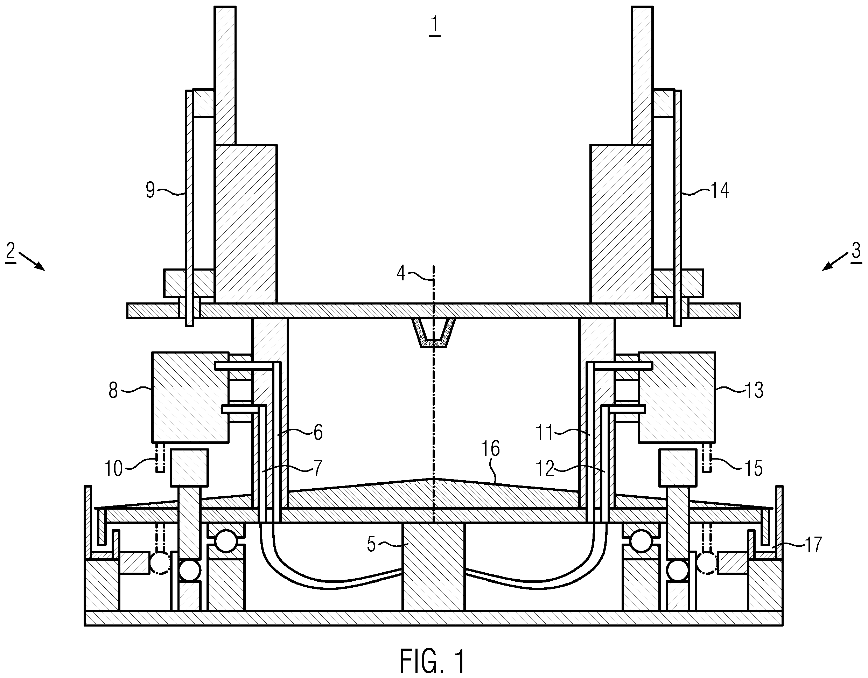

Die Figur 1 zeigt eine Schnittdarstellung der Vorrichtung 1 zur gleichzeitigen Herstellung von mehreren Fasern umfassenden Behältern unter der Verwendung von faserhaltiger Pulpe wobei in der Darstellung zwei Stationen 2, 3 zum jeweiligen Ausformen eines Fasern umfassenden Behälters zu sehen sind. Die Vorrichtung 1 kann insgesamt zwei, drei, vier oder mehr der Stationen 2, 3 umfassen. Die Vorrichtung 1 ist um eine Drehachse 4 drehbar ausgebildet, wobei die Drehachse senkrecht oder im Wesentlichen senkrecht verlaufend, beispielsweise zu einem Hallenboden, ausgerichtet ist. Die Stationen 2, 3 sind an einem Umfang der Vorrichtung 1 angeordnet. Der Umfang kann der Außenumfang sein oder ein anderer durch die Vorrichtung 1 beschreibbarer Umfang.

Die faserhaltige Pulpe kann mittels einer Vorrichtung 5 zum Zuführen von faserhaltiger Pulpe, hier beispielsweise ein Drehverteiler, einer Vor- bzw. Pressform 8, 13 der einzelnen Stationen 2, 3 gleichzeitig zugeführt werden. Dazu kann beispielsweise jeweils eine erste Rohrleitung 6, 11 und eine zweite Rohrleitung 7, 12 von der Vorrichtung 5 zum Zuführen von faserhaltiger Pulpe zu der Vor- bzw. Pressform 8, 13 der jeweiligen Station 2, 3 führen. Mittels der ersten Rohrleitung 6, 11 kann ein anderer Bereich der Vor- bzw. Pressform 8, 13 mit der faserhaltigen Pulpe versorgt werden als mittels der zweiten Rohrleitung 7, 12. Beispielsweise kann mittels der ersten Rohrleitung 6, 11 faserhaltige Pulpe ein Hals-Schulter-Bereich des herzustellenden, Fasern umfassenden Behälters mit faserhaltiger Pulpe versorgt werden und mittels der zweiten Rohrleitung 7, 12 beispielsweise der Körper-Boden-Bereich.

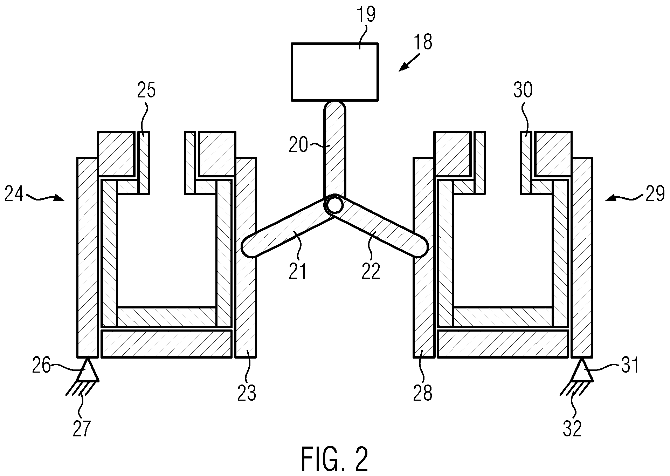

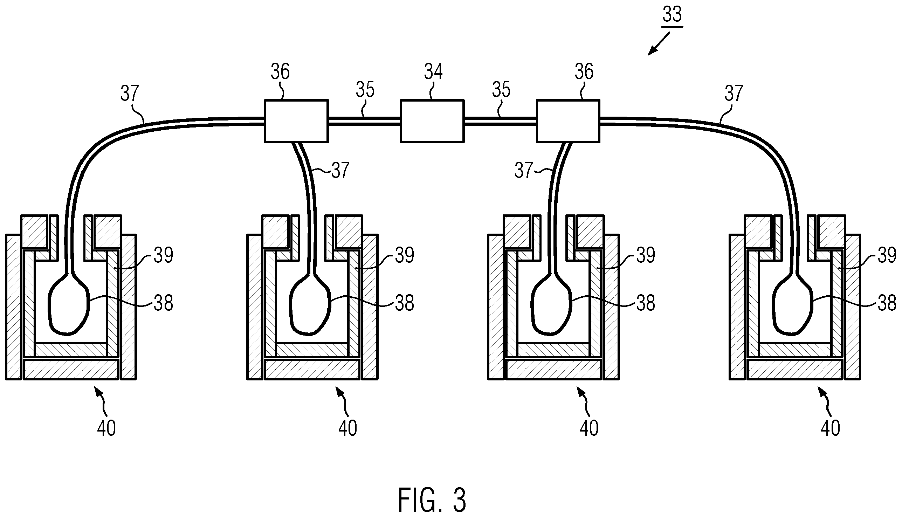

Die Vorrichtung 1 kann mindestens eine Übertragungsvorrichtung von Presskraft auf mindestens zwei der Pressformen umfassen, die hier nicht dargestellt sind, aber in den Figuren 2 und 3 beschrieben werden. Aufgrund der übertragenen Presskraft kann die an der Innenfläche der Vor- bzw. Pressform 8, 13 angelagerte faserhaltige Pulpe gepresst werden. Dabei kann zumindest ein Teil des Wassers ausgepresst werden, das in der faserhaltigen Pulpe enthalten sein kann. Dieser Teil des Wassers kann die Vor- bzw. Pressform 8, 13 über einen Auslass 10, 15 verlassen.

Um das aus dem Auslass austretende Wasser auffangen zu können, ist in Wirkrichtung der Schwerkraft unterhalb der Stationen 2, 3 eine Vorrichtung zum Sammeln und Rückführen des Wassers vorgesehen. Die Vorrichtung zum Sammeln und Rückführen des Wassers umfasst eine ringförmige Wanne 17 und eine zur Wanne 17 geneigte äußere Mantelfläche 16 eines Kreiskegels. Entlang der Schräge der äußeren Mantelfläche 16 kann das austretende Wasser zu der ringförmigen Wanne 17 fließen.

Die Vorrichtung kann mindestens einen Sprühkopf umfassen, durch den Reinigungsmedium in den Bereich der Stationen gespritzt werden kann. Das Reinigungsmedium kann im beispielsweise Wasser sein oder umfassen. Ein Sprühkopf ist beispielhaft in Figur 1 kurz unterhalb des Bezugszeichens 4 angeordnet.

Weiter kann die Vorrichtung 1, hier nicht dargestellt, eine Entnahmevorrichtung zum Entnehmen der Fasern umfassenden Behälter aus den Vor- bzw. Pressformen, eine Füllvorrichtung zum gleichzeitigen Befüllen der mehreren Fasern umfassenden Behälter mit Produkt, eine Verschließvorrichtung zum gleichzeitigen Verschließen der mehreren Fasern umfassenden Behälter und/oder eine Etikettiervorrichtung zum gleichzeitigen oder aufeinanderfolgenden Etikettieren der Fasern umfassenden Behälter umfassen. Bei einem Teil oder allen diesen Elementen kann es sich um separate Rundläufer handeln, die mit Transportsternen und/oder mit Transportbändern und/oder mit einem Langstator-Linearmotor-System untereinander verbunden sein können.

Die Figur 2 zeigt eine Schnittdarstellung einer ersten Ausführungsform einer Übertragungsvorrichtung 18 von Presskraft auf zwei Pressformen 24, 29. Die Übertragungsvorrichtung 18 umfasst einen Motor 19 und einen dreiarmigen Hebel 20, 21, 22, der durch den Motor 19 antreibbar ist. So kann auf eine erste Wand 23 der einen Pressform 24 und auf eine zweite Wand 28 der anderen Pressform 29 ein Druck ausgeübt werden. Auf an Innenflächen der Pressformen 24, 29 angelagerte, faserhaltige Pulpe 25, 30 kann dadurch Druck ausgeübt und jeweils ein Fasern umfassender Behälter ausgeformt werden. Wasser, das in der faserhaltigen Pulpe vorhanden ist, kann durch das Pressen zumindest teilweise ausgepresst werden. Dieser Anteil des Wassers 27, 32 kann die Pressformen 24, 29 durch vorgesehene Auslässe 26, 31 verlassen und kann möglicherweise für eine weitere Verwendung aufgefangen werden.

Die Figur 3 zeigt eine Schnittdarstellung einer zweiten Ausführungsform einer Übertragungsvorrichtung 33 von Presskraft auf vier Pressformen 40. In den Pressformen 40 ist an den Innenflächen jeweils faserhaltige Pulpe 39 angelagert und in Innern einer jeden Pressform 40 befindet sich ein elastischer Ballon 38, der mittels Druckluft aufblasbar ist. Im aufgeblasenen Zustand kann der elastische Ballon 38 die angelagerte faserhaltige Pulpe 39 an die Innenfläche der Pressform pressen, wodurch ein Fasern umfassender Behälter ausgeformt werden kann.

Die Druckluft wird in einer Bereitstellungsvorrichtung 34, beispielsweise einem Druckbehälter, bereitgestellt. Mittels zwei ersten Rohrleitungen 35 ist die Bereitstellungsvorrichtung 34 mit zwei Verteilern 36 verbunden. Je einer der Verteiler ist über zwei zweite Rohrleitungen 37 mit zwei der elastischen Ballone 38 verbunden. Für jeden der elastischen Ballone 38 kann ein Ventil zum Verschließen vorgesehen sein, um nach Einbringen der Druckluft diese für eine gegebene Zeitdauer in dem elastischen Ballon belassen zu können.

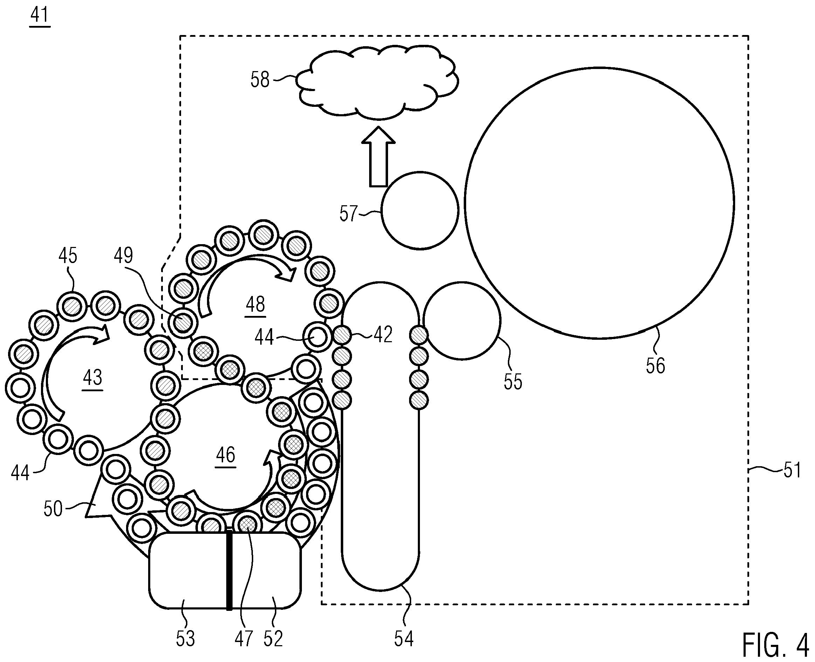

Die Figur 4 zeigt eine Draufsicht auf eine Vorrichtung 41 zur gleichzeitigen Herstellung von mehreren Fasern umfassenden Behältern 42 unter der Verwendung von faserhaltiger Pulpe.

Die Vorrichtung 41 umfasst ein erstes rundlaufendes Karussell 43 und die Stationen und somit die Vorformen und Pressformen sind an einem Umfang des ersten Karussells 43 angeordnet. Die leeren Vorformen und Pressformen sind mit dem Bezugszeichen 44 und ohne Füllung des Innenkreises kenntlich gemacht. Das erste Karussell 41 ist für ein Anströmen der faserhaltigen Pulpe in den Vorformen und Pressformen ausgebildet. Die Vorformen und Pressformen mit der angeströmten, Wasser umfassenden faserhaltigen Pulpe sind mit dem Bezugszeichen 45 und einer ersten Schraffierung des Innenkreises kenntlich gemacht. Mittels einer optionalen Heizvorrichtung können die Vorformen und Pressformen geheizt werden.

Die Vorformen und Pressformen 45 mit der angeströmten, Wasser umfassenden faserhaltigen Pulpe werden an ein zweites rundlaufendes Karussell 46 übergeben, beispielsweise mittels eines oder mehreren Transfersternen (die Teilungsverzugssterne umfassen können). Das zweite Karussell 46 ist stromab des ersten Karussells 43 angeordnet. Das zweite Karussell 46 ist für eine Innentrocknung von in der Vorform und Pressform 45 angeströmten faserhaltigen Pulpe ausgebildet. Die Innentrocknung kann mittels warmer Luft erfolgen. Die Vorformen und Pressformen, in denen die angeströmte, Wasser umfassende faserhaltige Pulpe von innen getrocknet wurde, sind mit dem Bezugszeichen 47 und einer zweiten Schraffierung des Innenkreises kenntlich gemacht. Da die Innentrocknung einige Zeit benötigt, sind die Vorformen und Pressformen, in denen die angeströmte, Wasser umfassende faserhaltige Pulpe von bereits innen getrocknet wurde, erst nach einem gewissen Umlauf auf dem zweiten Karussell 46 zu finden.

Die Vorformen und Pressformen 47, in denen die angeströmte, Wasser umfassende faserhaltige Pulpe von innen getrocknet wurde, werden an ein drittes rundlaufendes Karussell 48 übergeben, beispielsweise mittels eines oder mehreren Transfersternen (die Teilungsverzugssterne umfassen können). Das dritte Karussell 48 ist stromab des zweiten Karussells 46 angeordnet. Das dritte Karussell 48 ist für ein Pressen der angeströmten und von innen getrockneten faserhaltigen Pulpe und für ein Aufbringen einer Innenbeschichtung ausgebildet. Das dritte Karussell 48 ist in einem aseptischen Raum 51 angeordnet. Die Vorformen und Pressformen, in denen die angeströmte und von innen getrocknete faserhaltige Pulpe gepresst und eine Innenbeschichtung aufgebracht wurde, sind mit dem Bezugszeichen 49 und einer dritten Schraffierung des Innenkreises kenntlich gemacht.

Die Vorrichtung 41 umfasst weiter eine Rückführvorrichtung 50 zum Rückführen der Vorformen und Pressformen 44 nach Entnahme von Fasern umfassenden Behälter zu dem ersten Karussell 43. Die Fasern umfassenden Behälter können an einer Entnahmeposition des dritten Karussells 48 aus den Vorformen und Pressformen entnommen werden. Dazu können die Vorformen und Pressformen geöffnet werden.

Die Vorrichtung 41 umfasst zudem eine Reinigungsvorrichtung 52 und eine Inspektionsvorrichtung 53 für mittels der Rückführvorrichtung 50 rückgeführten Vorformen und Pressformen 44. Die Vorformen und Pressformen 44 können vor einem erneuten Verwenden durch das erste Karussell 43 gereinigt und inspiziert werden. Gegebenenfalls können defekte Vorformen und Pressformen 44 ausgeschleust werden.

Die Vorrichtung 41 umfasst weiter eine Trocknungsvorrichtung 54 für ein Trocknen von außen der aus den Vorformen und Pressformen entnommenen Fasern umfassenden Behälter 42. Die von außen getrockneten Behälter werden mittels eines Transfersterns 55 an eine Füllvorrichtung 56 übergeben und können dort mit Produkt befüllt werden. Von der Füllvorrichtung 56 werden die befüllten Behälter mittels eines Transfersterns 57 an eine Verschließvorrichtung 58 übergeben und können dort verschlossen werden. Die Trocknungsvorrichtung 54, die Füllvorrichtung 56 und die Verschließvorrichtung 58 sind in dem aseptischen Raum 51 angeordnet.

## Patentansprüche

1. Vorrichtung (1, 41) zur gleichzeitigen Herstellung von mehreren Fasern umfassenden Behältern unter der Verwendung von faserhaltiger Pulpe.

2. Die Vorrichtung nach Anspruch 1, umfassend mehrere Stationen (2, 3) zum jeweiligen Ausformen eines Fasern umfassenden Behälters, wobei beispielsweise jede Station (2, 3) eine Vorform und eine Pressform (24, 29, 40) umfasst, wobei beispielsweise die Vorform eine Gitterstruktur umfasst.

3. Die Vorrichtung nach einem der Ansprüche 1 bis 2, weiter umfassend eine Vorrichtung (5) zum Zuführen von faserhaltiger Pulpe, wobei beispielsweise die Vorrichtung zum Zuführen von faserhaltiger Pulpe einen Drehverteiler umfasst.

4. Die Vorrichtung nach einem der Ansprüche 1 bis 3, weiter umfassend eine Vorrichtung zum Sammeln und Rückführen zumindest eines Teils von Wasser, das in der faserhaltigen Pulpe umfasst ist, wobei beispielsweise die Vorrichtung zum Sammeln und Rückführen zumindest eines Teils von Wasser, das in der faserhaltigen Pulpe umfasst ist, den Drehverteiler umfasst,
wobei beispielsweise die Vorrichtung zum Sammeln und Rückführen zumindest eines Teils von Wasser, das in der faserhaltigen Pulpe umfasst ist, in Wirkrichtung der Schwerkraft unterhalb der mehreren Stationen (2, 3) angeordnet ist,
wobei beispielsweise die Vorrichtung zum Sammeln und Rückführen eine Wanne, beispielsweise eine ringförmige Wanne (17), beispielsweise als Wasserschloss ausgebildet, umfasst,
wobei beispielsweise zudem in Wirkrichtung der Schwerkraft unterhalb der mehreren Stationen (2, 3) eine zur Wanne geneigte äußere Mantelfläche (16) eines Kreiskegels vorgesehen ist.

5. Die Vorrichtung nach einem der Ansprüche 2 bis 4, weiter umfassend mindestens eine Übertragungsvorrichtung (18) von Presskraft auf mindestens zwei der Pressformen (24, 29),
wobei beispielsweise die mindestens eine Übertragungsvorrichtung (18) umfasst:
- einen Motor (19),
- mindestens einen Hebel (20, 21, 22), beispielsweise mindestens einen Kniehebel, der durch den Motor (19) antreibbar ist, wodurch auf die mindestens zwei der Pressformen (24, 25) ein Pressdruck ausübbar ist, und/oder
wobei beispielsweise die mindestens eine Übertragungsvorrichtung (33) umfasst:
- eine Bereitstellungsvorrichtung (34) für Druckluft,
wobei die Bereitstellungsvorrichtung (34) mit mindestens zwei elastischen Ballonen (38) mittels eines Rohrleitungssystems (35, 37) zum Verteilen der Druckluft verbunden ist, wobei jeder der elastischen Ballone (38) vorgesehen ist zum Einbringen in eine der Pressformen (40) und zum Aufblasen mittels der Druckluft.

6. Die Vorrichtung nach einem der Ansprüche 1 bis 5, wobei die Vorrichtung (1) ausgebildet ist, bei der gleichzeitigen Herstellung der mehreren Fasern umfassenden Behälter eine trennbare Verbindung der mehreren Behälter unter Verwendung der faserhaltigen Pulpe vorzusehen,
wobei beispielsweise die trennbare Verbindung eine oder mehrere Perforationen umfasst, wobei beispielsweise die trennbare Verbindung Stege und/oder eine durchgehende flächige Verbindung zwischen den mehreren Behältern umfasst.

7. Die Vorrichtung nach einem der Ansprüche 1 bis 6, weiter umfassend eine Entnahmevorrichtung zum Entnehmen der Fasern umfassenden Behälter aus den Vor- bzw. Pressformen, beispielsweise umfassend eine Füllvorrichtung zum gleichzeitigen Befüllen der mehreren Behälter mit Produkt, beispielsweise weiter umfassend eine Verschließvorrichtung zum gleichzeitigen Verschließen der mehreren Behälter, beispielsweise weiter umfassend eine Etikettiervorrichtung zum gleichzeitigen oder aufeinanderfolgenden Etikettieren der Behälter.

8. Die Vorrichtung nach einem der Ansprüche 4 bis 7, weiter umfassend eine Vorrichtung zum Herstellen der faserhaltigen Pulpe, wobei die Vorrichtung zum Herstellen der faserhaltigen Pulpe mit der Vorrichtung zum Sammeln und Rückführen zumindest eines Teils von Wasser, das in der faserhaltigen Pulpe umfasst ist, durch eine Zuführleitung leitungsverbunden ist zum Zuführen des zumindest einen Teils von Wasser, das in der faserhaltigen Pulpe umfasst ist, und zum Verwenden des zumindest einen Teils von Wasser, das in der faserhaltigen Pulpe umfasst ist, für die Herstellung weiterer faserhaltiger Pulpe,
wobei beispielsweise die Zuführleitung eine Desinfektionsvorrichtung umfasst zum Desinfizieren des zumindest einen Teils von Wasser, das in der faserhaltigen Pulpe umfasst ist, wobei beispielsweise die Desinfektionsvorrichtung ausgebildet ist zum Ausbringen eines Desinfektionsmittels in den zumindest einen Teils von Wasser, das in der faserhaltigen Pulpe umfasst ist, und/oder zum Ausbringen von UV-A-, UV-B- und/oder UV-C-Strahlung zum Bestrahlen des zumindest einen Teils von Wasser, das in der faserhaltigen Pulpe umfasst ist.

9. Die Vorrichtung nach Anspruch 8, weiter umfassend eine Regelungsvorrichtung zum Messen einer Qualität des zumindest einen Teils von Wasser, das in der faserhaltigen Pulpe umfasst ist, und zum Regeln der Desinfektionsvorrichtung entsprechend eines Ergebnisses der Messung der Qualität.

10. Die Vorrichtung nach einem der Ansprüche 2 bis 9, wobei die Vorrichtung (41) ein rundlaufendes Karussell (43) umfasst und wobei die Stationen (2, 3) an einem Umfang des rundlaufenden Karussells (43) vorgesehen sind, wobei das rundlaufende Karussell (43) für ein Anströmen der faserhaltigen Pulpe in der Vorform und Pressform (44) ausgebildet ist, wobei beispielsweise jeder der Vorformen und Pressformen (44) ein Einlassventil zugeordnet ist, wobei beispielsweise eine Heizvorrichtung für die Vorformen und Pressformen (44) vorgesehen ist.

11. Die Vorrichtung nach Anspruch 10, wobei die Vorrichtung (41) ein zweites rundlaufendes Karussell (46) umfasst, das stromab des rundlaufenden Karussells (43) angeordnet ist, wobei das zweite rundlaufende Karussell (46) für eine Innentrocknung von in der Vorform und Pressform angeströmten faserhaltigen Pulpe ausgebildet ist, und/oder,
wobei die Vorrichtung (41) ein drittes rundlaufendes Karussell (48) umfasst, das stromab des rundlaufenden Karussells (43) angeordnet ist, wobei das dritte rundlaufende Karussell (48) für ein Pressen von in der Vorform und Pressform angeströmten faserhaltigen Pulpe und für ein Aufbringen einer Innenbeschichtung ausgebildet ist, wobei beispielsweise das dritte rundlaufende Karussell (48) in einem aseptischen Raum (51) angeordnet ist.

12. Die Vorrichtung nach Anspruch 11, wobei das rundlaufende Karussell weiter für ein Pressen von in der Vorform und Pressform angeströmten faserhaltigen Pulpe ausgebildet ist.

13. Die Vorrichtung nach Anspruch 11 oder 12, weiter umfassend eine Rückführvorrichtung (50) zum Rückführen der Vorformen und Pressformen nach Entnahme von Fasern umfassenden Behälter zu dem rundlaufenden Karussell (43), beispielsweise weiter umfassend eine Reinigungsvorrichtung (52) und eine Inspektionsvorrichtung (53) für mittels der Rückführvorrichtung rückgeführten Vorformen und Pressformen, wobei beispielsweise die Inspektionsvorrichtung (53) eine Ausschleusvorrichtung für defekte Vorformen und Pressformen umfasst.

14. Die Vorrichtung nach einem der Ansprüche 11 bis 13, wobei die Vorrichtung (41) eine Trocknungsvorrichtung (54) für ein Trocknen von außen der aus den Vorformen und Pressformen entnommenen Fasern umfassenden Behälter (42), eine Füll- und Verschließvorrichtung (56, 58) und eine Etikettiervorrichtung umfasst, wobei beispielsweise die Trocknungsvorrichtung (54) und die Füll- und Verschließvorrichtung (56, 58) in einem aseptischen Raum (51) angeordnet sind.

15. Die Vorrichtung nach einem der Ansprüche 1 bis 4 und 7 bis 14, wobei die Vorrichtung (1, 41) derart ausgebildet ist, dass die Fasern umfassenden Behälter derart anordenbar sind, dass eine Längsachse der Behälter zu einer Wirkrichtung der Schwerkraft einen Winkel zwischen 70° bis 110° einschließt.

16. Die Vorrichtung nach einem der Ansprüche 1 bis 15, wobei die Vorrichtung (1, 41) modular aufgebaut ist.

17. Verfahren zur gleichzeitigen Herstellung von mehreren Fasern umfassenden Behältern unter der Verwendung von faserhaltiger Pulpe mittels der Vorrichtung (1, 41) nach einem der Ansprüche 1 bis 16.
